# EUROPEAN PATENT SPECIFICATION

(11) **EP 2 726 510 B1**
(45) Date of publication and mention of the grant of the patent: **08.03.2023**
(21) Application number: 12725311.0
(22) Date of filing: 29.05.2012
(51) Int. Cl.: C07K 16/46, C07K 16/12, C07K 16/22, C07K 16/24, C07K 16/40, C12N 15/10

(54) **DUAL TARGETING**
ZWEIFACHES TARGETING
CIBLAGE DOUBLE

(30) Priority: 27.05.2011 EP 11004373
(43) Date of publication of application: 07.05.2014
(73) Proprietor: F. Hoffmann-La Roche AG, 4070 Basel (CH)
(72) Inventor: BECKMANN, Roland, A-1190 Vienna (AT); JENSEN, Hobolt Kristian, 1190 Vienna (AT)
(74) Representative: Mertes, Maria Margot Martha
(86) International application number: PCT/EP2012/002279
(87) International publication number: WO 2012/163520

(56) References cited:
- WO-A2-03/002609
- WO-A2-2008/027236
- BOSTROM JENNY ET AL: "Variants of the antibody herceptin that interact with HER2 and VEGF at the antigen binding site", SCIENCE, AMERICAN ASSOCIATION FOR THE ADVANCEMENT OF SCIENCE, WASHINGTON, DC; US, vol. 323, no. 5921, 20 March 2009 (2009-03-20), pages 1610-1614, XP002560553, ISSN: 0036-8075, DOI: 10.1126/SCIENCE.1165480
- GABRIELE SCHAEFER ET AL: "A Two-in-One Antibody against HER3 and EGFR Has Superior Inhibitory Activity Compared with Monospecific Antibodies", CANCER CELL, CELL PRESS, US, [Online] vol. 20, no. 4, 18 October 2011 (2011-10-18), pages 472-486, XP002679896, ISSN: 1535-6108, DOI: 10.1016/J.CCR.2011.09.003 Retrieved from the Internet: URL:http://www.sciencedirect.com/science/a rticle/pii/S1535610811003515> [retrieved on 2011-10-17]
- JENNY BOSTROM ET AL: "High Affinity Antigen Recognition of the Dual Specific Variants of Herceptin Is Entropy-Driven in Spite of Structural Plasticity", PLOS ONE, vol. 6, no. 4, 1 January 2011 (2011-01-01) , pages e17887-e17887, XP55038984, ISSN: 1932-6203, DOI: 10.1371/journal.pone.0017887
- IGAWA TOMOYUKI ET AL: "Engineering the variable region of therapeutic IgG antibodies", MABS, LANDES BIOSCIENCE, US, vol. 3, no. 3, 1 May 2011 (2011-05-01), pages 243-252, XP009153597, ISSN: 1942-0870

## Description

### FIELD OF THE INVENTION

The present invention relates to antibody-based dual targeting molecules, and to methods for generating such dual targeting molecules, including a library-based approach.

### BACKGROUND OF THE INVENTION

This invention relates to a design for bispecific antibodies or functional frágments thereof as defined in the appended claims.

In the literature various approaches to generating bispecific antibody molecules have been reported. These approaches can be divided into two categories: 1) generating bispecific antibody formats in which the two paratopes recognizing two targets or two epitopes both lie within one heterodimeric antibody variable region formed by one complementary VH-VL pair and both comprise CDR residues belonging to this complementary VH-VL pair, and 2) generating other bispecific antibody formats in which the two paratopes recognizing two targets or epitopes do not both lie within one heterodimeric antibody variable region formed by one complementary VH-VL pair and do not both comprise CDR residues belonging to the same complementary VH-VL pair.

Within the first category of approaches, only two methods of predictably engineering bi-specific antibody molecules have been described in the literature, and these will be discussed in detail below in Sections [0014] to [0015]. However, to put this work into context, the second category of approaches will be summarized first.

This second category of approaches (in which the two paratopes recognizing two targets or epitopes do not both lie within one heterodimeric antibody variable region formed by one complementary VH-VL pair and do not both comprise CDR residues belonging to the same complementary VH-VL pair) constitutes a very large body of work by various previous workers, and numerous diverse examples of such bi-specific antibodies have been described.

In a first group of examples belonging to the second category of approaches, two or more antibody fragments (including Fab fragments, single chain Fvs, or single domain antibodies) of different specificities are combined by chemical linkage or by genetic fusion via one or more peptide linkers. Published bi-specific antibody formats in this group of examples include the following:
a. Diabodies (Perisic et al., Structure. 1994 Dec 15;2(12):1217-26; Kontermann, Acta Pharmacol Sin. 2005 Jan;26(1):1-9; Kontermann, Curr Opin Mol Ther. 2010 Apr;12(2):176-83.)
b. TandAbs etc. (Cochlovius et al., Cancer Res. 2000 Aug 15;60(16):4336-41.)
c. Single domains specific to different targets genetically fused by peptide linkers (e.g. Domantis: WO2008/096158; Ablynx: WO2007/112940)
d. Others (for reviews, see: Enever et al., Curr Opin Biotechnol. 2009 Aug;20(4):405-11. Epub 2009 Aug 24.; Carter, Nat. Rev. Immunol. 6, 343 (2006); P. Kufer et al., Trends Biotechnol. 22, 238 (2004)).

To improve their potential usability in medical applications, the *in vivo* serum half-life of the above bi-specific antibody formats can be extended using various technologies, including the following:
a. Addition of serum albumin or a serum albumin binding entity
b. PEGylation
c. Addition of a protein polymer by genetic fusion, such as HAPylation (Schlapschy et al., Protein Eng Des Sel. 2007 Jun;20(6):273-84. Epub 2007 Jun 26) or XTEN (Schellenberger, Nat. Biotechnology 12 (2009) 1186).

In this group of examples, the bispecific antibodies comprised of antibody fragments lack an Fc region and therefore generally do not show the natural binding to the neonatal Fc receptor FcRn, do not exhibit the natural effector functions (ADCC and CDC, ref.) of full IgG antibodies, and can usually not be purified via superantigen-derived affinity resins, such as protein A resins specific for the Fc region, in an identical manner to IgG antibodies. These consequences of lack of an Fc region can limit the achievable serum half-life, the feasible applications as active drug ingredients and the economic manufacturing of such bispecific antibodies.

In a second group of examples belonging to the second category of approaches, bispecific antibodies comprise an IgG-like molecule and one or several additional appended binding domains or entities. Such antibodies include IgG-scFv fusion proteins in which a single chain Fv has been fused to one of the termini of the heavy chains or light chains (University of California, Biogen Idec, CAT/Medlmmune), and dual variable domain (dvd-lgG) molecules in which an additional VH domain and a linker are fused to the N-terminus of the heavy chain and an additional VL domain and a linker are fused to the N-terminus of the light chain (Abbott). In general these approaches suffer from disadvantages in terms of manufacturing, accessibility, and stability of the constructs.

In a third group of examples belonging to the second category of approaches, bispecific antibodies comprise IgG-like antibodies that have been generated or modified in such a way that they exhibit two specificities without the addition of a further binding domain or entity. Such antibodies include IgG molecules in which the naturally homodimeric CH3 domain has been modified to become heterodimeric, e.g. using an engineered protuberation (Ridgway et al., Protein Eng. 1996 Jul;9(7):617-21), using strand exchange (Davis et al., Protein Eng Des Sel. 2010 Apr;23(4):195-202. Epub 2010 Feb 4), or using engineered opposite charges (Novo Nordisk), thereby potentially enabling the two halves of the IgG-like molecule to bind two different targets through the binding entities added to the Fc region, usually N-terminal Fab regions. Antibodies in this third group of examples also include IgG molecules in which some structural loops not naturally involved in antigen contacts are modified to bind a further target in addition to one bound naturally through variable region CDR loops, for example by point mutations in the Fc region (e.g. Xencor Fcs binding to FcgRllb) or by diversification of structural loops (e.g. f-star Mab2 with diversified CH3 domain). These approaches suffer from disadvantages in terms of stability, manufacturing, valency, and limited affinity/applications.

In contrast to all of the above examples of bi-specific antibodies in the second category, bi-specific antibodies in the first category have two paratopes specific for two targets which both comprise CDR residues located within the same heterodimeric VH-VL antibody variable region. Only four types of antibody molecules attributable to this first category have been described in the art. Of these four types, the first type of antibody is not truly bi-specific as it cannot specifically recognize two unrelated targets; the second type of antibody occurs naturally but it is not known whether it can be predictably engineered as no example of such work is published; and only the third and fourth types of antibody can be engineered with specificity towards two unrelated targets according to publications. The four types of antibody molecules attributable to the first category are the following:

Cross-reactive antibodies, which have a single broad specificity that corresponds to two or more structurally related antigens or epitopes. For such antibodies the two antigens are related in sequence and structure. For example, antibodies may cross-react with related targets from different species, such as hen egg white lysozyme and turkey lysozyme (WO 92/01047) or with the same target in different states or formats, such as hapten and hapten conjugated to carrier (Griffiths AD et al. EMBO J 1994 13: 14 3245-60). It is possible to deliberately engineer antibodies for cross-reactivity. For example, antibodies have been engineered to recognize two related antigens from different species (example Genentech: antibody binding human LFA1 engineered to also bind rhesus LFA1, resulting in successful drug Raptiva/Efalizumab). Similarly, WO 02/02773 describes antibody molecules with "dual specificity". The antibody molecules referred to are antibodies raised or selected against multiple structurally related antigens, with a single binding specificity that can accommodate two or more structurally related targets. However, as mentioned above, all these cross-reactive antibodies are not truly bi-specific and are not engineered to specifically recognize two unrelated targets.

Furthermore, there are polyreactive autoantibodies, which occur naturally (Casali & Notkins, Ann. Rev. Immunol. 7, 515-531). These polyreactive antibodies have the ability to recognize at least two (usually more) different antigens or epitopes that are not structurally related. It has also been shown that selections of random peptide repertoires using phage display technology on a monoclonal antibody will identify a range of peptide sequences that fit the antigen-binding site. Some of the sequences are highly related, fitting a consensus sequence, whereas others are very different and have been termed mimotopes (Lane & Stephen, Current Opinion in Immunology, 1993,5, 268-271). It is therefore clear that the binding sites of some heterodimeric VH-VL antibodies have the potential to bind to different and sometimes unrelated antigens. However, as mentioned above, such polyreactive antibodies may be found but have not been deliberately engineered using predictable methods described in the art.

One method described in the art that allows the deliberate engineering of bi-specific antibodies able to bind two structurally unrelated targets through two paratopes, both residing within one complementary heterodimeric VH-VL pair and both comprising CDR residues belonging to this complementary VH-VL pair, relates to "two-in-one" antibodies. These "two-in-one" antibodies are engineered to comprise two overlapping paratopes using methods somewhat distinct from previous cross-reactivity-engineering methods. This work has been described in WO 2008/027236 and by Bostrom et al. (Bostrom et al., Science. 2009 Mar 20;323(5921):1610-4). In the published examples, a heterodimeric VH-VL antibody variable region specific for one target (HER2) was isolated and thereafter the light chain was re-diversified to achieve additional specificity for a second target (VEGF or death receptor 5). For one of the resulting antibodies the binding was characterized by structure resolution and it was found that 11 out of 13 VH and VL CDR residues making contact with HER2 in one antibody-antigen complex also made contact with VEGF in the alternative antibody-antigen complex. While the published "two-in-one" antibodies retained nanomolar affinities for HER2, only one of the clones published by Bostrom et al. (2009) had a nanomolar affinity of 300 nM for the additional target, VEGF, while four other clones had micromolar affinities for the additional targets. It is clear that while this approach has achieved binding to two structurally unrelated targets, a degree of surface compatibility between the two targets is needed to enable the specificities of two overlapping paratopes. It also has not been described in detail how highly specific such "two-in-one" antibodies are for only two targets, and whether some general non-specific binding or "stickiness" of such antibodies, potentially caused by the need for some conformational flexibility of side chains located in the overlapping portion of the two paratopes, can be observed.

A second method described in the art that allows the deliberate engineering of bi-specific antibodies able to bind two structurally unrelated targets through two paratopes, both residing within one complementary heterodimeric VH-VL pair and both comprising CDR residues belonging to this complementary VH-VL pair, relates to antibodies comprising complementary pairs of single domain antibodies. WO 2003/002609 and US 2007/026482 have described heterodimeric VH-VL antibodies, in which a heavy chain variable domain recognizes one target and a light chain variable domain recognizes a second structurally unrelated target, and in which the two single domains with different specificities are combined into one joint heterodimeric VH-VL variable region. In the published examples of such antibodies, the single domains were first separately selected as an unpaired VH domain or as an unpaired VL domain to bind the two unrelated targets, and afterwards combined into a joint heterodimeric VH-VL variable region specific to both targets.

For all molecules belonging to the first category of bispecific antibodies (able to bind two targets through two paratopes, both residing within one complementary heterodimeric VH-VL pair and both comprising CDR residues belonging to this complementary VH-VL pair), no additional domains or entities need to be fused to an IgG molecule, no structural loops of an IgG molecule need to be diversified and no limiting hetero-bi-specific Fc regions need to be utilized in order to achieve the dual specificity. This has several potential benefits:

The risk of reducing protein stability is reduced because no structural loops have to be diversified and no constant domain interfaces have to be modified, resulting in potentially greatly improved biophysical properties of the antibodies.

No potentially easily proteolysed or potentially immunogenic linkers are required, resulting in an improved developability of the antibodies as active drug ingredients.

No undesirable pairings of VH and VL domains can occur, avoiding potential byproducts comprising mispaired heterodimeric VH-VL variable regions during expression, because only one unique VH region and one unique VL region is required.

No reduced expression or formation of unusual covalent aggregates are expected, because no additional disulphide bonds are required compared to conventional monospecific antibodies.

The bi-specific heterodimeric variable regions comprising two paratopes within one complementary heterodimeric VH-VL pair can be combined with different constant domains, including Fc regions. This offers several advantages:
a. Potentially improved manufacturing using fully established methods, for example methods identical to those used in the manufacturing of conventional mono-specific IgGs.
b. FcRn-mediated serum-half-life modulation in patients and animal models.
c. Free choice of effector functions associated with different isotypes, ranging from non-cytotoxic, essentially inert behavior (for example in antibodies designed for receptor blockade) to aggressive cytotoxic behavior (for example in antibodies designed to kill tumor cells).

The above third example of "two-in-one" antibodies derived by methods related to cross-reactivity engineering is potentially greatly limited in its medical applicability by competition of the two unrelated targets for the overlapping, at least partially shared binding residues within the CDR loops. Furthermore, the inherently sequential selection process of "two-in-one" antibodies, with specificity first achieved for one target, followed by re-diversification and then discovery of clones specific for an additional target, is time-consuming and unpredictable, because only a limited number of antibodies specific for the first target can be re-diversified into selectable libraries but it is unknown which of the first specific clones will be most amenable to engineering the additional desired specificity. Finally, the isolation and affinity maturation of "two-in-one" antibodies is severely complicated by the fact that any improvement of variable domain sequences to increase binding to one target can potentially cause a reduction in affinity for the other target.

The above fourth example of binding one target through light chain CDR loop residues and another target through heavy chain CDR loop residues is severely complicated by the fact that some of the potentially important light chain CDR residues responsible for binding to the first target are directly adjacent to some of the potentially important heavy chain CDR residues responsible for binding to the second target in the final, packed, bi-specific heterodimeric antibody variable region. This means that in its bound state, the first target recognized by such antibodies can potentially compete with the second target recognized by such antibodies due to steric hindrance, thereby potentially limiting the medical applicability of such antibodies. Furthermore, if light chains and heavy chains of such antibodies are isolated independently by selection and screening methods as was described in the historic example of US2007026482 (Abbott Laboratories), combining them into bi-specific antibodies may potentially affect the affinities of the originally independent domains towards the individual targets in the combined bi-specific molecules due to conformational changes in the CDRs that could potentially occur upon pairing of heavy and light chains. Finally, combining pre-isolated VH and VL variable domains with a variety of CDR loops is likely to result in unpredictable antibody stability, as it has been described by Wörn and Plückthun (1998) and Röthlisberger et al. (2005) that important interactions and a mutual stabilization of antibody heavy and light chains occur between VH and VL domains.

Conversely, the bispecific, heterodimeric variable regions comprising two paratopes within one complementary heterodimeric VH-VL pair could be used as antibody fragments such as Fab fragments or single chain Fvs and would not require the presence of an Fc region to achieve their dual specificity, allowing the option of microbial manufacturing in the absence of mammalian N-glycosylation mechanisms, and their use in therapeutic or diagnostic applications where a low molecular weight or short serum half-life are desirable.

Thus, while the approach of having two paratopes within one complementary heterodimeric VH-VL pair offers so many advantages, the attempts pursued so far, which have been described above, have had limited success.

Thus, there is still a large unmet need to provide an improved format for the bispecific antibodies that incorporates the advantages of having two paratopes within one complementary heterodimeric VH-VL pair, while avoiding the problems observed with the prior art constructs.

The solution for this problem that has been provided by the present invention, i.e. the design of two paratopes for each complementary heterodimeric VH-VL pair, wherein each paratope uses residues from CDR regions from both VH and VL domains, has so far not been achieved or suggested by the prior art.

### SUMMARY OF THE INVENTION

The present invention relates to bispecific antibodies characterized by having two paratopes for each complementary heterodimeric VH-VL pair, wherein each paratope uses residues from CDR regions from both VH and VL domains, as specified in the appended claims.

Thus, in a first aspect, the present invention relates to an antibody or functional fragment thereof comprising at least one variable binding domain consisting of a heavy chain variable (VH) domain and a light chain variable (VL) domain, wherein said binding domain comprises two paratopes for two unrelated epitopes, wherein (i) binding of each paratope to its epitope does not prevent the simultaneous binding of the other paratope to its respective epitope, and wherein (ii) both paratopes comprise at least one residue from at least one VH CDR and at least one residue from at least one VL CDR.

In a second aspect, the present invention relates to nucleic acid sequence encoding the antibody or functional fragment thereof according to the present invention.

In a third aspect, the present invention relates to a vector comprising the nucleic acid sequence according to the present invention.

In a fourth aspect, the present invention relates to a host cell comprising the nucleic acid sequence according to the present invention, or the vector according to the present invention.

In a fifth aspect, the present invention relates to a method for generating the antibody or functional fragment thereof according to the present invention, comprising the step of expressing the nucleic acid sequence according to the present invention, or the vector according to the present invention, either *in vitro* or from an appropriate host cell, including the host cell according to the present invention.

Also described is a collection of antibodies or functional fragment thereof, wherein said collection comprises a diverse collection of antibody variable domain sequences wherein either (i) at least 3 CDR residues from Lib1 positions are diversified, provided that at least one diversified residue is located within the VH domain and at least one diversified position is located within the VL domain, and wherein no residues from Lib2 positions are diversified, or (ii) at least 3 CDR residues from Lib2 positions are diversified, provided that at least one diversified residue is located within the VH domain and at least one diversified position is located within the VL domain, and wherein no residues from Lib1 positions are diversified.

Also described is a method of generating a bispecific antibody molecule or functional fragment thereof comprising the steps of
a. generating a first collection of antibody molecules or functional fragment thereof, each comprising a heterodimeric VH-VL variable region, with diversity in at least 3 CDR positions selected from the group of Lib1, provided that at least one diversified residue is located within the VH domain and at least one diversified position is located within the VL domain, and wherein no residues from Lib2 positions are diversified;
b. selecting a first antibody molecule or functional fragment thereof specific for a first target or epitope from said first collection;
c. generating a second collection of antibody molecules or functional fragment thereof, each comprising a heterodimeric VH-VL variable region, with diversity in at least 3 CDR positions selected from the group of Lib2, provided that at least one diversified residue is located within the VH domain and at least one diversified position is located within the VL domain, and wherein no residues from Lib1 positions are diversified;
d. selecting a second antibody molecule or functional fragment thereof specific for a second target or epitope from said second collection; and
e. generating a nucleic acid sequence that encodes a third antibody molecule or functional fragment thereof comprising a heterodimeric VH-VL variable region, wherein the third antibody molecule or functional fragment thereof comprises at least 3 residues found in the group of Lib1 positions in the first antibody molecule or functional fragment thereof, of which at least one residue is located within the VH domain and at least one residue is located within the VL domain, and wherein the third antibody molecule or functional fragment thereof further comprises at least 3 residues found in the group of Lib2 positions in the second antibody molecule or functional fragment thereof, of which at least one residue is located within the VH domain and at least one residue is located within the VL domain.

Also described is a method of generating a bispecific antibody molecule or functional fragment thereof comprising the steps of
a. generating a first collection of antibody molecules or functional fragment thereof, each comprising a heterodimeric VH-VL variable region, with diversity in at least 3 CDR positions selected from the group of Lib1, provided that at least one diversified residue is located within the VH domain and at least one diversified position is located within the VL domain, and wherein no residues from Lib2 positions are diversified;
b. selecting a first antibody molecule or functional fragment thereof specific for a first target or epitope from said first collection;
c. generating a second collection of antibody molecules or functional fragment thereof, each comprising a heterodimeric VH-VL variable region, by diversifying said first antibody molecule of functional fragment thereof by introducing diversity in at least 3 CDR positions selected from the group of Lib2, provided that at least one diversified residue is located within the VH domain and at least one diversified position is located within the VL domain, and wherein no residues from Lib1 positions are diversified; and
d. selecting a second antibody molecule or functional fragment thereof specific for said first and a second target or epitope from said second collection; and
e. alternatively, performing steps a. to d. with the modification that the first collection in step a. is generated by diversifying at least 3 CDR positions selected from the group of Lib2, and diversifying in step c. said first antibody or antibody fragment thereof in at least 3 CDR positions selected from the group of Lib1.

In a sixth aspect, the present invention relates to pharmaceutical compositions comprising an antibody molecule or functional fragment thereof, and optionally a pharmaceutically acceptable carrier and/or excipient.

### BRIEF DESCRIPTION OF THE DRAWINGS

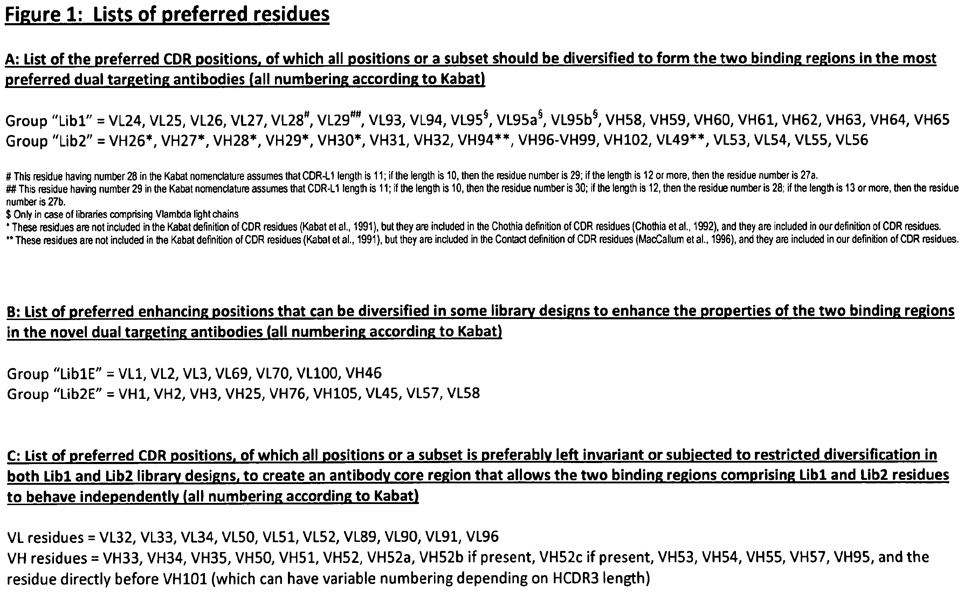

**Figure 1** below shows the list of preferred CDR positions of which all or a subset should be diversified in antibody libraries in some embodiments of our present invention (A), the list of preferred optional enhancing positions in the framework regions which may also be diversified in antibody libraries in some embodiments of the invention (B), and the list of CDR positions of which all or a subset are preferably left invariant in all libraries of the present invention, i.e. both in libraries in which Lib1 residues are diversified and in libraries in which Lib2 residues are diversified (C).

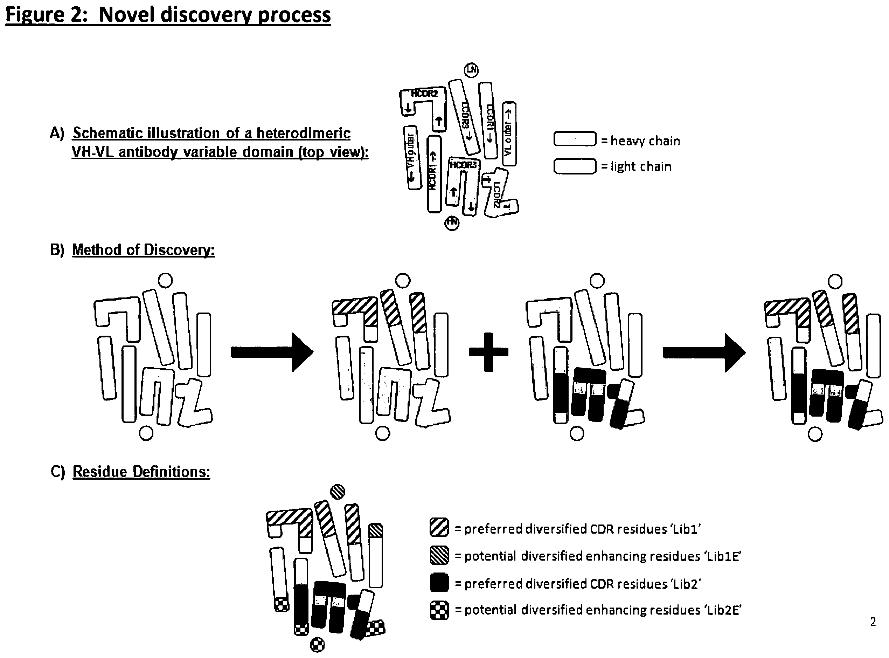

**Figure 2** below illustrates in a schematic way the discovery process of the novel bi-specific antibodies, using the top view (aerial) perspective to show how a heterodimeric VH-VL antibody scaffold is first diversified separately in two regions representing Lib1 and Lib2 CDR residues; this yields two libraries that are separately selected to obtain two antibody clones, with one clone binding a first target or epitope via a first paratope and the second clone binding a second target or epitope via a second paratope; these clones are then combined into a bi-specific antibody according to the present invention, by introducing target-specific residues selected in Lib1 positions in the first antibody clone into the second antibody clone, or by introducing target-specific residues selected in Lib2 positions in the second antibody clone into the first antibody clone. Figure 2 also illustrates in a schematic way the location of those potential enhancing residues according to the current invention in the framework regions that are visible from the top view (aerial) perspective.

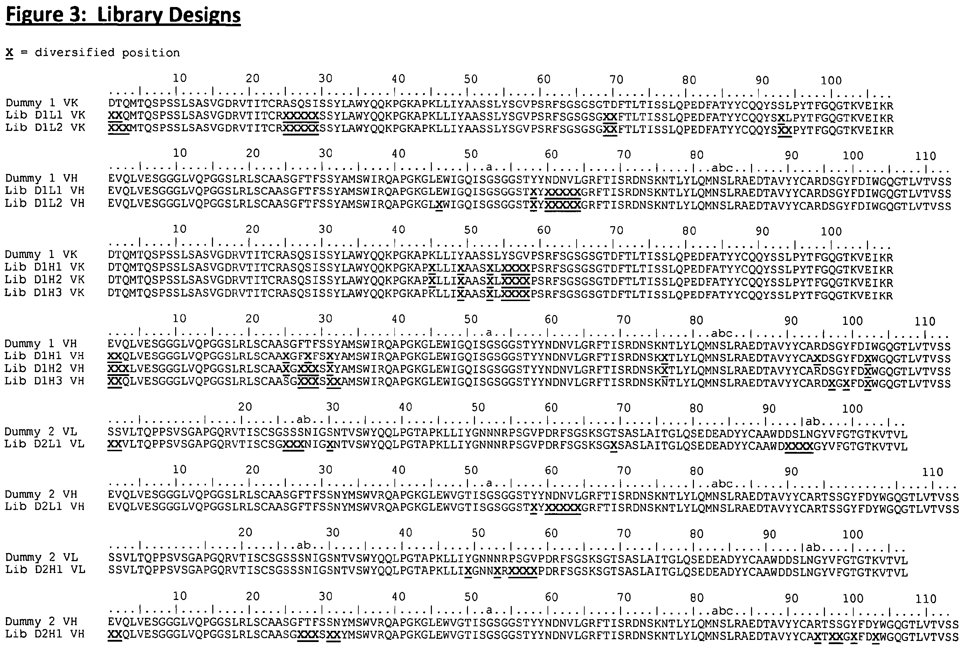

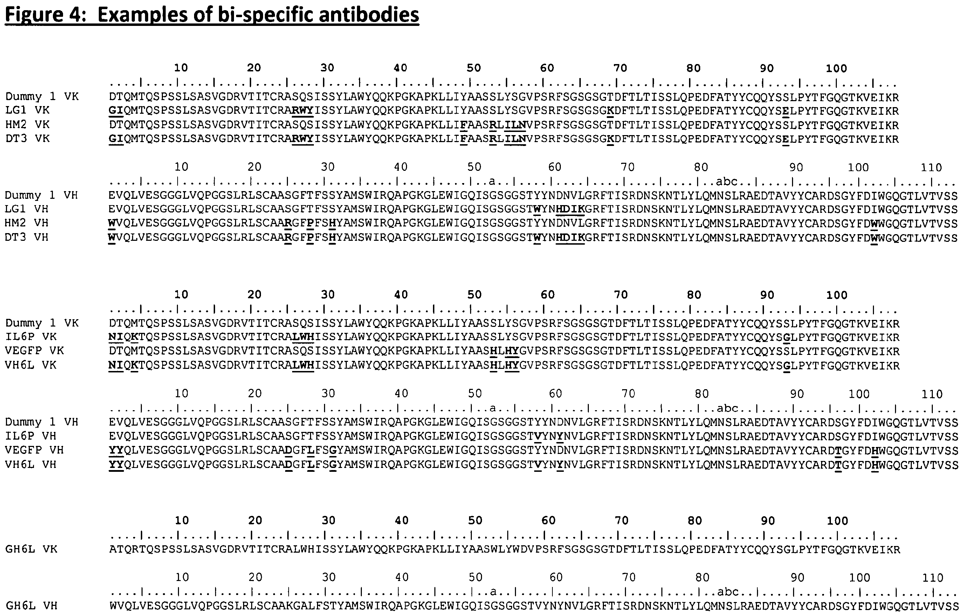

**Figure 3** shows four preferred library designs (libraries Lib D1L1, Lib D1L2, Lib D1H1 and Lib D1H2), which we have tested. We have produced each of these four libraries as a pool of synthetic genes encoding human Fab fragments with the shown VH3-VK1 pairing as heterodimeric VH-VL scaffold. The synthetic genes in each library were constant in the positions for which a specific amino acid is displayed in the single letter code, and diversified in the positions marked by "X". The four libraries were each produced as phage display libraries and sorted against several antigens using standard methods known in the art. Selected antibody clones from these four libraries have been combined into the bi-specific antibodies detailed in Figure 4. Figure 3 further shows three additional preferred library designs (Lib D1H3, Lib D2L1 and Lib D2H1).
**Figure 4** gives examples of sequences of bi-specific antibodies, which were generated according to the present invention.
**Figure 5** shows the specificity of the antibodies disclosed in Figure 4, demonstrated by ELISA analysis of an anti-MBP anti-GST dual targeting clone HM2LG1.

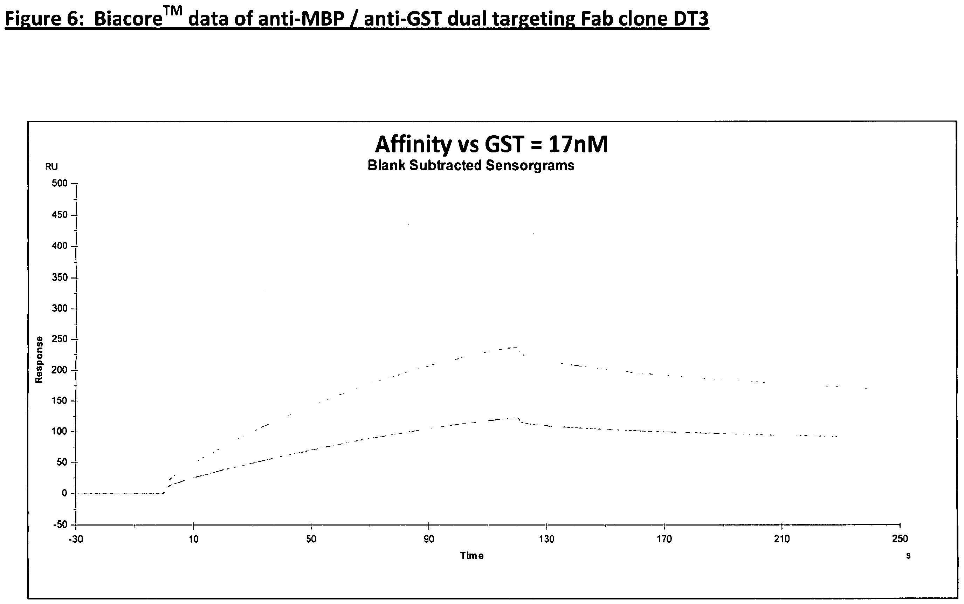

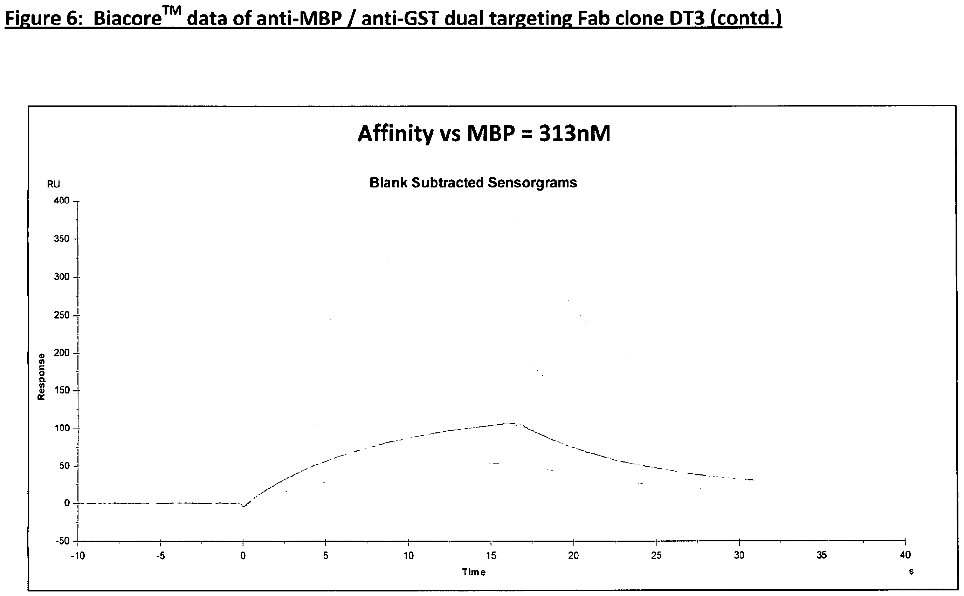

**Figure 6** shows Biacore^{™} data illustrating the high specificity of bispecific constructs according to the invention.
**Figure 7** shows a Biacore^{™} analysis of parental and bi-specific antibodies against VEGF and IL6.
**Figure 8** shows Biacore^{™} data illustrating the independent co-binding of two targets to a bi-specific construct according to the invention: **A:** co-binding of GMCSF + Antibody + IL6; **B:** co-binding of anti-LC + Antibody + IL6

### DETAILED DESCRIPTION OF THE INVENTION

The peculiarity of this invention compared to former approaches for the construction of bispecific antibodies is the so far unknown possibility to have two paratopes for each complementary heterodimeric VH-VL pair, wherein each paratope uses residues from CDR regions from both VH and VL domains.

Thus, in a first aspect, the present invention relates to an antibody or functional fragment thereof comprising at least one variable binding domain consisting of a heavy chain variable (VH) domain and a light chain variable (VL) domain, wherein said binding domain comprises two paratopes for two unrelated epitopes, wherein (i) binding of each paratope to its epitope does not prevent the simultaneous binding of the other paratope to its respective epitope, and wherein (ii) both paratopes comprise at least one residue from at least one VH CDR and at least one residue from at least one VL CDR.

As used herein, the term "antibody" refers to an immunoglobulin (Ig) molecule that is defined as a protein belonging to the class IgG, IgM, IgE, IgA, or IgD (or any subclass thereof), which includes all conventionally known antibodies and functional fragments thereof. A "functional fragment" of an antibody/immunoglobulin molecule hereby is defined as a fragment of an antibody/immunoglobulin molecule (e.g., a variable region of an IgG) that retains the antigen-binding region. An "antigen-binding region" of an antibody typically is found in one or more hypervariable region(s) (or complementarity-determining region, "CDR") of an antibody molecule, i.e. the CDR-1, -2, and/or - 3 regions; however, the variable "framework" regions can also play an important role in antigen binding, such as by providing a scaffold for the CDRs. Preferably, the "antigen-binding region" comprises at least amino acid residues 4 to 103 of the variable light (VL) chain and 5 to 109 of the variable heavy (VH) chain, more preferably amino acid residues 3 to 107 of VL and 4 to 111 of VH, and particularly preferred are the complete VL and VH chains (amino acid positions 1 to 109 of VL and 1 to 113 of VH; numbering according to WO 97/08320). A preferred class of antibody molecules for use in the present invention is IgG.

"Functional fragments" of the invention include the domain of a F(ab')2 fragment, a Fab fragment, scFv or constructs comprising single immunoglobulin variable domains or single domain antibody polypeptides, e.g. single heavy chain variable domains or single light chain variable domains. The F(ab')2 or Fab may be engineered to minimize or completely remove the intermolecular disulphide interactions that occur between the CH1 and CL domains.

An antibody may be derived from immunizing an animal, or from a recombinant antibody library, including an antibody library that is based on amino acid sequences that have been designed *in silica* and encoded by nucleic acids that are synthetically created. *In silico* design of an antibody sequence is achieved, for example, by analyzing a database of human sequences and devising a polypeptide sequence utilizing the data obtained therefrom. Methods for designing and obtaining *in* silico-created sequences are described, for example, in Knappik et al., J. Mol. Biol. (2000) 296:57; Krebs et al., J. Immunol. Methods. (2001) 254:67; and U.S. Pat. No. 6,300,064 issued to Knappik et al.

In the context of the present invention, the term "bispecific antibody molecule" refers to an antibody molecule, including a functional fragment of an antibody molecule, that comprises specific binding sites for two different target biomolecules, or two different epitopes, either present on one target biomolecule, or present on two different molecules, such as on the target biomolecule and a second biomolecule.

As used herein, a binding molecule is "specific to/for", "specifically recognizes", or "specifically binds to" a target, such as a target biomolecule (or an epitope of such biomolecule), when such binding molecule is able to discriminate between such target biomolecule and one or more reference molecule(s), since binding specificity is not an absolute, but a relative property. In its most general form (and when no defined reference is mentioned), "specific binding" refers to the ability of the binding molecule to discriminate between the target biomolecule of interest and an unrelated biomolecule, as determined, for example, in accordance with specificity assay methods known in the art. Such methods comprise, but are not limited to Western blots, ELISA, RIA, ECL, IRMA tests and peptide scans. For example, a standard ELISA assay can be carried out. The scoring may be carried out by standard colour development (e.g. secondary antibody with horseradish peroxide and tetramethyl benzidine with hydrogenperoxide). The reaction in certain wells is scored by the optical density, for example, at 450 nm. Typical background (= negative reaction) may be about 0.1 OD; typical positive reaction may be about 1 OD. This means the ratio between a positive and a negative score can be 10-fold or higher. Typically, determination of binding specificity is performed by using not a single reference biomolecule, but a set of about three to five unrelated biomolecules, such as milk powder, BSA, transferrin or the like.

In the context of the present invention, the term "about" or "approximately" means between 90% and 110% of a given value or range.

However, "specific binding" also may refer to the ability of a binding molecule to discriminate between the target biomolecule and one or more closely related biomolecule(s), which are used as reference points. Additionally, "specific binding" may relate to the ability of a binding molecule to discriminate between different parts of its target antigen, e.g. different domains, regions or epitopes of the target biomolecule, or between one or more key amino acid residues or stretches of amino acid residues of the target biomolecule.

In the context of the present invention, the term "paratope" refers to that part of a given antibody molecule that is required for specific binding between a target and the antibody molecule. A paratope may be continuous, i.e. formed by adjacent amino acid residues present in the antibody molecule, or discontinuous, i.e. formed by amino acid residues that are at different positions in the primary sequence of the amino acid residues, such as in the amino acid sequence of the CDRs of the amino acid residues, but in close proximity in the three-dimensional structure, which the antibody molecule adopts.

In the context of the present invention, the term "epitope" refers to that part of a given target that is required for specific binding between the target and an antibody. An epitope may be continuous, i.e. formed by adjacent structural elements present in the target, or discontinuous, i.e. formed by structural elements that are at different positions in the primary sequence of the target, such as in the amino acid sequence of a protein as target, but in close proximity in the three-dimensional structure, which the target adopts in a native environment, such as in a bodily fluid.

In one embodiment, the antibody or functional fragment thereof is a bispecific antibody.

In further embodiments of the antibody or functional fragment of the present invention, the amount of binding of each paratope to its respective epitope in the simultaneous presence of both epitopes is at least 25% of the amount of binding that is achieved in the absence of the other epitope under otherwise identical conditions.

In further embodiments of the antibody or functional fragment of the present invention, the amount of binding is at least 50%, particularly at least 75%, and more particularly at least 90%.

In further embodiments of the antibody or functional fragment of the present invention, the first paratope comprises residues from CDR1 and CDR3 of the VL domain and CDR2 of the VH domain, and the second paratope comprises residues from CDR1 and CDR3 of the VH domain and CDR2 of the VL domain.

In particular embodiments, the antibody or functional fragment thereof is a human antibody or functional fragment thereof.

In further embodiments, the antibody or functional fragment of the present invention is based on a human VH3 family heavy chain sequence and a human Vkappa1 family light chain sequence.

In further embodiments, the antibody or functional fragment of the present invention is based on a human VH3 family heavy chain sequence and a human Vlambda1 family light chain.

In further embodiments, the antibody or functional fragment of the present invention is selected from a single chain Fv fragment, a Fab fragment and an IgG.

In further embodiments of the antibody or functional fragment thereof of the invention, binding to one epitope can be knocked out by mutating one of the Lib1 or Lib2 positions, while binding to the other epitope is kept intact.

In this context, the phrase "binding ..[is] .. knocked out" refers to a situation where the affinity to the epitope is reduced at least 10-fold (e.g. from 1 nM to 10 nM), and the phrase "binding ..is kept intact" refers to a situation where the affinity to the epitope is reduced at maximum 3-fold (e.g. from 1 nM to 3 nM).

In particular such embodiments, binding to one epitope can be knocked out by mutating one of the positions VL position 27 or VH position 61, or by mutating one of the Lib2 positions VL position 56 or VH position 28.

In particular such embodiments, binding to one epitope can be knocked out by mutating one of the residues listed in section [0066] to R, when the residue is selected from D, N, E and Q, or by mutating such residue to D, when the residue is different from D, N, E or Q.

In a further aspect, the present invention relates to a binding molecule comprising at least one antibody variable domain comprising one variable light chain and one variable heavy chain, wherein said antibody variable domain is binding to at least a first and a second target, wherein binding of said antibody variable domain to said first target is independent from binding of said antibody variable domain to said second target and *vice versa,* and wherein said first and second target are neither anti-idiotypic antibodies, nor non-physiological peptides, such as peptides used for epitope mapping.

In the context of the present invention, binding of the antibody variable domain to one target is "independent" from binding to the other target, when the amount of binding of the first paratope to its respective epitope (the first target) in the simultaneous presence of both targets is at least 25% of the amount of binding that is achieved in the absence of the other target under otherwise identical conditions. In particular, the amount of binding is at least 50%, particularly at least 75%, and more particularly at least 90%.

In particular embodiments, said first and said second target are both physiologically relevant targets and/or epitopes thereof, including disease-related targets, such as cancer-related antigens, cell surface receptors, cytokines and/or other signaling molecules.

In a second aspect, the present invention relates to nucleic acid sequence encoding the antibody or functional fragment thereof according to the present invention.

In a third aspect, the present invention relates to a vector comprising the nucleic acid sequence according to the present invention.

In a fourth aspect, the present invention relates to a host cell comprising the nucleic acid sequence according to the present invention, or the vector according to the present invention.

In a fifth aspect, the present invention relates to a method for generating the antibody or functional fragment thereof according to the present invention, comprising the step of expressing the nucleic acid sequence according to the present invention, or the vector according to the present invention, either *in vitro* or from an appropriate host cell, including the host cell according to the present invention.

Also described is a collection of antibodies or functional fragment thereof, wherein said collection comprises a diverse collection of antibody variable domain sequences wherein either (i) at least 3 CDR residues from Lib1 positions are diversified, provided that at least one diversified residue is located within the VH domain and at least one diversified position is located within the VL domain, and wherein no residues from Lib2 positions are diversified, or (ii) at least 3 CDR residues from Lib2 positions are diversified, provided that at least one diversified residue is located within the VH domain and at least one diversified position is located within the VL domain, and wherein no residues from Lib1 positions are diversified.

Also described in the context of the previous paragraph is a collection of antibodies or functional fragment thereof, wherein in the case of (i) at least one residue of each of CDR1 and CDR3 of the VL domain and CDR2 of the VH is diversified, or in the case of (ii) at least one residue of each of CDR1 and CDR3 of the VH domain and CDR2 of the VL is diversified.

Also described in the context of paragraph 76 is a collection of antibodies or functional fragment thereof, wherein in the case of (i) at least one residue of the Lib1E positions in said variable binding domain is additionally diversified, and/or wherein in the case of (ii) at least one residue of the Lib2E positions in said variable binding domain is additionally diversified.

Also described is a method of generating a bispecific antibody molecule or functional fragment thereof comprising the steps of
a. generating a first collection of antibody molecules or functional fragment thereof, each comprising a heterodimeric VH-VL variable region, with diversity in at least 3 CDR positions selected from the group of Lib1, provided that at least one diversified residue is located within the VH domain and at least one diversified position is located within the VL domain, and wherein no residues from Lib2 positions are diversified;
b. selecting a first antibody molecule or functional fragment thereof specific for a first target or epitope from said first collection;
c. generating a second collection of antibody molecules or functional fragment thereof, each comprising a heterodimeric VH-VL variable region, with diversity in at least 3 CDR positions selected from the group of Lib2, provided that at least one diversified residue is located within the VH domain and at least one diversified position is located within the VL domain, and wherein no residues from Lib1 positions are diversified;
d. selecting a second antibody molecule or functional fragment thereof specific for a second target or epitope from said second collection; and
e. generating a nucleic acid sequence that encodes a third antibody molecule or functional fragment thereof comprising a heterodimeric VH-VL variable region, wherein the third antibody molecule or functional fragment thereof comprises at least 3 residues found in the group of Lib1 positions in the first antibody molecule or functional fragment thereof, of which at least one residue is located within the VH domain and at least one residue is located within the VL domain, and wherein the third antibody molecule or functional fragment thereof further comprises at least 3 residues found in the group of Lib2 positions in the second antibody molecule or functional fragment thereof, of which at least one residue is located within the VH domain and at least one residue is located within the VL domain.

Also described is a method of generating a bispecific antibody molecule or functional fragment thereof comprising the steps of
a. generating a first collection of antibody molecules or functional fragment thereof, each comprising a heterodimeric VH-VL variable region, with diversity in at least 3 CDR positions selected from the group of Lib1, provided that at least one diversified residue is located within the VH domain and at least one diversified position is located within the VL domain, and wherein no residues from Lib2 positions are diversified;
b. selecting a first antibody molecule or functional fragment thereof specific for a first target or epitope from said first collection;
c. generating a second collection of antibody molecules or functional fragment thereof, each comprising a heterodimeric VH-VL variable region, by diversifying said first antibody molecule or functional fragment thereof by introducing diversity in at least 3 CDR positions selected from the group of Lib2, provided that at least one diversified residue is located within the VH domain and at least one diversified position is located within the VL domain, and wherein no residues from Lib1 positions are diversified;
d. selecting a second antibody molecule or functional fragment thereof specific for said first and a second target or epitope from said second collection; and
e. alternatively, performing steps a. to d. with the modification that the first collection in step a. is generated by diversifying at least 3 CDR positions selected from the group of Lib2, and diversifying in step c. said first antibody or antibody fragment thereof in at least 3 CDR positions selected from the group of Lib1.

Also described in the context of paragraphs 79 and 80 is a method, further comprising the step of:
f. expressing the nucleic acid sequence generated in steps a. to e. in a host cell or translating the nucleic acid into protein representing the third antibody molecule or functional fragment thereof.

Also described is a method, wherein any of said collection having diversity selected from group Lib1 includes additional diversity in at least one enhancing position selected from the group of Lib1E and/or wherein any of said collection having diversity selected from group Lib1 includes additional diversity in at least one enhancing position selected from the group of Lib2E.

Also described is a method, wherein said first collection is identical to a library selected from Lib D1L1, Lib D1L2 and Lib D2L1, or is derived from such a library having the diversified positions present in Lib D1L1, Lib D1L2 or Lib D2L1 in combination with more than 90% sequence identity, particularly more than 95% sequence identity, in the framework regions; and wherein said first collection is identical to a library selected from Lib D1H1, Lib D1H2, Lib D1H3 and Lib D2H1, or is derived from such a library having the diversified positions present in Lib D1H1, Lib D1H2, Lib D1H3 or Lib D2H1 in combination with more than 90% sequence identity, particularly more than 95% sequence identity, in the framework regions.

In certain such embodiments, the antibody molecule or functional fragment thereof is selected from a single chain Fv fragment, a Fab fragment and an IgG.

In a ninth aspect, the present invention relates to pharmaceutical compositions comprising an antibody molecule or functional fragment thereof, and optionally a pharmaceutically acceptable carrier and/or excipient. The compositions may be formulated e.g. for once-a-day administration, twice-a-day administration, or three times a day administration.

The phrase "pharmaceutically acceptable", as used in connection with compositions of the invention, refers to molecular entities and other ingredients of such compositions that are physiologically tolerable and do not typically produce untoward reactions when administered to a mammal (e.g., human). The term "pharmaceutically acceptable" may also mean approved by a regulatory agency of the Federal or a state government or listed in the U.S. Pharmacopeia or other generally recognized pharmacopeia for use in mammals, and more particularly in humans.

In the context of the present invention, the term "about" or "approximately" means between 90% and 110% of a given value or range.

The term "carrier" applied to pharmaceutical compositions of the invention refers to a diluent, excipient, or vehicle with which an active compound (e.g., a bispecific antibody fragment) is administered. Such pharmaceutical carriers may be sterile liquids, such as water, saline solutions, aqueous dextrose solutions, aqueous glycerol solutions, and oils, including those of petroleum, animal, vegetable or synthetic origin, such as peanut oil, soybean oil, mineral oil, sesame oil and the like. Suitable pharmaceutical carriers are described in "Remington's Pharmaceutical Sciences" by A.R. Gennaro, 20th Edition.

The active ingredient (e.g., a bispecific antibody fragment) or the composition of the present invention may be used for the treatment of at least one disease or disorder, wherein the treatment is adapted to or appropriately prepared for a specific administration as disclosed herein (e.g., to once-a-day, twice-a-day, or three-times-a-day administration). For this purpose the package leaflet and/or the patient information contains corresponding information.

The active ingredient (e.g., the bispecific antibody molecule or fragment thereof) or the composition of the present invention may be used for the manufacture of a medicament for the treatment of at least one disease or disorder, wherein the medicament is adapted to or appropriately prepared for a specific administration as disclosed herein (e.g., to once-a-day, twice-a-day, or three-times-a-day administration). For this purpose the package leaflet and/or the patient information contains corresponding information.

### EXAMPLES

The following examples illustrate the invention without limiting its scope.

While the first category of bi-specific antibody molecules described above (with two paratopes specific for two targets which both comprise CDR residues located within the same heterodimeric VH-VL antibody variable region) offers a range of potential benefits as described above, we hypothesized that an entirely novel class of antibody molecule could be created, that belongs to this first category of antibody molecules but is entirely different from the above-mentioned four examples that have been reported in the literature. We hypothesized that by pursuing an entirely novel approach, it might be possible to achieve some dramatic improvements in the deliberate engineering of antibodies belonging to this first category, compared to the examples mentioned above. This hypothesis took into account the fact that the historic methods mentioned above have some significant potential limitations in the development of antibodies as active drug ingredients.

According to the present invention, we describe an entirely novel class of bi-specific antibodies, which address these issues and have unexpected and dramatic advantages. We speculated that it may be possible to engineer two distinct paratopes within the VH-VL variable region of a heterodimeric antibody, each comprising CDR residues from both the heavy chain and the light chain, but not overlapping and preferably not immediately adjacent to each other, in order to avoid conformational changes in one binding site as a result of mutations in the other binding site, and in order to reduce the likelihood of competition between the two targets in binding to the antibody (by minimizing possible steric hindrance between the two targets in their bound state). We further speculated that this novel class of antibody molecule could be engineered by first creating two synthetic antibody libraries, each in the background of a packed heterodimeric VH-VL pair, in one of which a first set (Lib1) of heavy and light chain CDR positions could be diversified and in the other one of which a different, non-overlapping set (Lib2) of heavy and light chain CDR positions could be diversified. We concluded that if such libraries could be created and successfully selected in parallel against two unrelated targets, then bi-specific antibodies could potentially be created rapidly by introducing the specific residues selected in the Lib1 positions during selections against the first target, into an antibody clone with specific residues selected in the Lib2 positions during selections against the second target. Vice versa, we also concluded that if such libraries could be created and successfully selected against two distinct targets, then bi-specific antibodies could potentially be created by introducing the residues selected in the Lib2 positions during selections against the second target into an antibody clone with specific residues selected in the Lib1 positions during selections against the first target. We speculated that this strategy of introducing a set of residues from a first antibody, defining a first specificity, into a second antibody of a second specificity would be greatly helped by creating both libraries within an identical or highly similar scaffold defining the packed VH-VL pair.

In the present application we demonstrate that we have successfully implemented this invention, creating several bi-specific heterodimeric VH-VL antibodies against two completely unrelated targets. Importantly, the antibodies were rapidly created and were highly specific for only two targets, showing no binding to additional unrelated targets. Surprisingly, the created bispecific antibodies showed not only a high biophysical stability (that has not been demonstrated for antibodies binding one target through light chain CDR loop residues and another target through heavy chain CDR loop residues), but an extremely high biophysical stability even compared to the scaffold used in the creation of "two-in-one" antibodies and compared to established monospecific antibody clones used as active ingredients in marketed drugs. Finally and also surprisingly, using the example of a bi-specific antibody against GM-CSF and TNF-alpha, we were able to demonstrate that a single conservative point mutation in a CDR position within the Lib1 binding region providing the putative paratope involved in TNF-alpha-binding essentially abolished binding to TNF-alpha whilst leaving binding to GM-CSF intact, and that a different single conservative point mutation in a CDR position within the Lib2 binding region providing the putative paratope involved in GM-CSF-binding completely abolished binding to GM-CSF whilst leaving binding to TNF-alpha intact. This demonstrates that the antibodies of our current invention can indeed bind two unrelated targets in a highly specific manner, rather than through general "stickiness", and that in contrast to above bi-specific antibodies known in the art, the two binding sites that are designed as non-overlapping paratopes are essentially independently behaved, although both are located in one heterodimeric VH-VL variable region and although both comprise CDR residues belonging to the same heterodimeric variable region. The antibodies of the present invention therefore have key advantages over prior bi-specific antibodies.

In preferred embodiments of the present invention, the preferred discovery process comprises the steps of (1) generating a pair of libraries based on the same or a highly similar heterodimeric VH-VL antibody scaffold by diversification of different CDR positions in the first and second library, (2) optionally also including diversification of selected framework positions in the VH-VL scaffold in one or both of the two libraries to potentially enhance the binding properties of clones selected from the two libraries, (3) selecting both libraries independently against two target molecules or epitopes and characterizing binders to identify target- or epitope-specific antibody clones with desired properties, (4) introducing all of the residues or a subset of the residues (preferably the majority of residues but no less than 3 of the residues) selected in diversified positions in an antibody clone selected from one library and specific for a first target or epitope into a target-specific antibody clone selected from the other library and specific for a second target or epitope. For this discovery process to work optimally, some groups of key residues play an important role:

By examining molecular models of heterodimeric VH-VL antibodies *in silico* and by performing mutagenesis of unselected heterodimeric VH-VL antibody "dummies" with no specificity (data not shown), we derived a list of CDR residues that could potentially be diversified to form the first potential binding site against the first target (Lib1 residues) and a list of CDR residues that could potentially be diversified to form the second potential binding site against the second target (Lib2 residues). We also derived a list of potential enhancing residues in the antibody framework regions, which in the folded antibody molecule are in close proximity to the Lib1 or Lib2 CDR residues and which can potentially be diversified to modify the properties and enhance the binding of the first paratope comprising Lib1 CDR residues to a first target (Lib1 E enhancing residues) and the binding of the second paratope comprising Lib2 CDR residues to a second target (Lib2E enhancing residues). Finally, we derived a list of CDR residues that would preferably be left identical or very similar in both libraries, to maintain an invariant packing of a central core region of the antibody molecule in both libraries, which would then also be present in all combined bi-specific antibody clones comprising a set of target-1-specific Lib1 and optionally Lib1E residues as well as a set of target-2-specific Lib2 and optionally Lib2E residues. We concluded that this invariant packed core region would shield the two binding sites from each other, making the first paratope against the first target somewhat immune to detrimental conformational effects resulting from changes in the second paratope against the second target. Indeed we have been able to demonstrate that the affinities and binding kinetics of parental antibody clones are usually closely matched by combined bi-specific antibody clones derived from the parental clones. Example 8 illustrates this using the exemplary antigens VEGF and IL6 where parental antibodies IL6P with an affinity of 38 nM and VEGFP with an affinity of 11 nM were combined to yield the bi-specific antibody VH6L with an affinity of 40 nM for IL6 and 7.8 nM for VEGF. This surprisingly high level of independence of the two binding sites makes it possible to affinity-mature them and in parallel in a way not possible for "two-in-one" antibodies (third historic example above) or bi-specific paired single domain heterodimers (fourth historic example above). We also concluded that the invariant core region may achieve a spacing between the two binding sites, potentially allowing them to bind two targets independently without competition caused by overlapping paratopes or by steric hindrance between a first bound and a second unbound target, depending on the nature and molecular size of each target molecule. Indeed, using the exemplary antigens GMCSF and IL6, we have been able to demonstrate for the novel class of bi-specific antibody molecules according to the invention that for some of the combined clones, co-binding of both antigens to a single VH-VL variable region is possible. Moreover, Example 9 illustrates that the affinity of the co-binding of the second antigen to the variable region can be independent of whether the first target is present or absent. The possibility of achieving such co-binding to the same VH-VL variable region and the possible independence of co-binding affinities have not been demonstrated for other types of historic bi-specific antibodies and represent a unique advantage of the novel antibodies according to the present invention. In some of the novel bi-specific antibodies, the independent binding behavior can further be demonstrated by mutations like those listed in Example 10. In such antibodies, it is possible to knock out or greatly reduce affinity for a first target whilst leaving affinity for a second target intact by making a point mutation in a Lib1 position, and *vice versa,* knock out or greatly reduce affinity for said second target whilst leaving affinity for said first target intact by making a point mutation in a Lib2 position.

### EXAMPLE 1: Construction of libraries

The synthetic gene pools for libraries Lib D1L1 and Lib D1H1 were purchased from GeneArt, while the synthetic gene pools for libraries D1L2 and D1H2 were purchased from Sloning Biotechnology. All four libraries were cloned into a newly constructed phage display vector which we built from the backbone pUC19 (that was purchased from NEB) by the addition of an M13 origin; two synthetic ribosome binding sites driving expression of antibody heavy and light chains; and synthetic genes encoding two signal peptides driving secretion of antibody polypeptides into the E. *coli* periplasm, human CH1 and CK constant domains and a truncated C-terminal portion of M13 protein III fused to the C-terminus of the human CH1 constant domain. The libraries were transformed into TG1 E. *coli* cells to yield 4 libraries with transformed diversities of 109 each. From the transformed TG1 *E. coli* cells, the four libraries were produced as libraries of phages displaying diversified Fab fragments, using M13KO7 helper phage and standard molecular biology methods as described by (Barbas et al., Phage Display: A Laboratory Manual, Cold Spring Harbour Laboratory Press, 1st ed., 2001; Sambrook, Molecular Cloning: A Laboratory Manual, Cold Spring Harbour Laboratory Press, 3rd ed., 2001).

### EXAMPLE 2: Panning

Binders from libraries of Fab-on-phage particles can be selected in accordance with standard panning procedures (Barbas et al., Phage Display: A Laboratory Manual, Cold Spring Harbour Laboratory Press, 1st ed., 2001; Sambrook, Molecular Cloning: A Laboratory Manual, Cold Spring Harbour Laboratory Press, 3rd ed., 2001) against immobilized targets MBP and GST.

### EXAMPLE 3: Screening

Phage particles selected in Example 2 can be rescued by infecting bacterial host cells (Barbas et al., Phage Display: A Laboratory Manual, Cold Spring Harbour Laboratory Press, 1st ed., 2001; Sambrook, Molecular Cloning: A Laboratory Manual, Cold Spring Harbour Laboratory Press, 3rd ed., 2001). Fab protein is expressed from individual clones and tested for specific binding against the targets MPB and GST. Positive hits are used in the next step to clone bispecific constructs.

### EXAMPLE 4: Cloning of bi-specific antibodies

Antibody genes were designed based on the desired amino acid sequence and purchased as synthetic genes or synthetic gene fragments from GeneArt or DNA2.0. Genes encoding antibody variants with point mutations were generated by PCR or overlap PCR, using the polymerase Pwo Master, purchased from Roche, and synthetic oligonucleotides encoding the desired point mutations, purchased from Thermo Fisher Scientific, according to manufacturer's instructions. An *E*. *coli* Fab expression vector was generated by modification of the plasmid pUC19, which was purchased from New England Biolabs. The pUC19 backbone was modified by the addition of two synthetic ribosome binding sites driving expression of antibody heavy and light chains, two synthetic signal peptide sequences driving the secretion of antibody chains into the *E*. *coli* periplasm and one M13 phage origin potentially enabling single strand production. Synthetic antibody genes, synthetic fragments of antibody genes and PCR-generated variants of antibody genes encoding point mutations were cloned into this *E*. *coli* Fab expression vector by restriction digestion, using restriction endonucleases purchased from Roche, followed by ligation, using LigaFast purchased from Promega, according to manufacturer's instructions. Ligation reactions were transformed into competent TG1 *E. coli* cells purchased from Stratagene or Zymoresearch.

### EXAMPLE 5: Antibody expression and purification

TG1 *E. coli* clones bearing Fab expression constructs were grown in LB and TB solid and liquid media, purchased from Carl Roth, which were supplemented with Carbenicillin and glucose, purchased from VWR. Antibody expression in liquid cultures was performed overnight in Erlenmeyer flasks in a shaking incubator and was induced by the addition of isopropyl-β-D-thiogalactopyranoside (IPTG), purchased from Carl Roth, to the growth medium. Culture supernatants containing secreted Fab fragments were clarified by centrifugation of the expression cultures. Clarified culture supernatants were supplemented with a 1% volume of Streptomycin/Penicillin solution, purchased from PAA Laboratories, a 2% volume of 1M Tris pH8.0, purchased from VWR, and a 0.4% volume of STREAMLINE rProtein A resin, purchased from GE Healthcare. The supplemented culture supernatants were incubated on a rolling incubator for 3 hours or overnight to achieve binding of Fab fragments to the protein A resin. Resins were then transferred into gravity flow columns, washed once using 30 bed volumes of 2x PBS pH 7.4, purchased from Invitrogen, washed once using 5 bed volumes of a buffer containing 10mM Tris pH 6.8 and 100 mM NaCl, purchased from VWR, and eluted using a buffer containing 10mM citric acid pH3 and 100mM NaCl, purchased from VWR. Eluted Fab fragments were neutralized by adding an 8% volume of 1M Tris pH 8.0. Neutralized purified Fab fragments were buffer exchanged into pure 1x PBS pH 7.4 (containing 1.06 mM KH₂PO₄, 2.97 mM Na₂HPO₄-7H₂O, 155.17 mM NaCl and no other supplements; Invitrogen catalogue No. 10010056), using illustra NAP-5 desalting columns from GE Healthcare, according to manufacturer's instructions.

### EXAMPLE 6: Antibody stability measurement

The biophysical stability of purified, buffer-exchanged Fab fragments was determined in 1x PBS pH 7.4 (Invitrogen catalogue No. 10010056) using differential scanning calorimetry (DSC). For all measurements, a capillary cell microcalorimeter equipped with autosampler and controlled by VPViewer2000 CapDSC software from MicroCal was used. All Fab fragments were scanned against pure buffer containing no antibody (1x PBS pH 7.4; Invitrogen catalogue No. 10010056). The scan parameters were set to analyze a temperature window from 32°C to between 105°C and 115°C, with a pre-scan thermostat of 2 minutes, a post-scan thermostat of 0 minutes and no gain. The scan rate was set to 250°C per hour for screening applications and to 60°C per hour for re-analysis of the most stable combination mutants. The absolute melting temperature of the Fab fragments determined in screening mode (scan-rate 250°C per hour) was 3.7°C to 4.5°C higher than in re-analysis mode (scan-rate 60°C per hour), but ranking of clones was the same in both modes. Melting temperatures of Fab fragments were determined after PBS reference subtraction, using Origin 7.0 software from MicroCal.

### EXAMPLE 7: Antibody specificity measurement

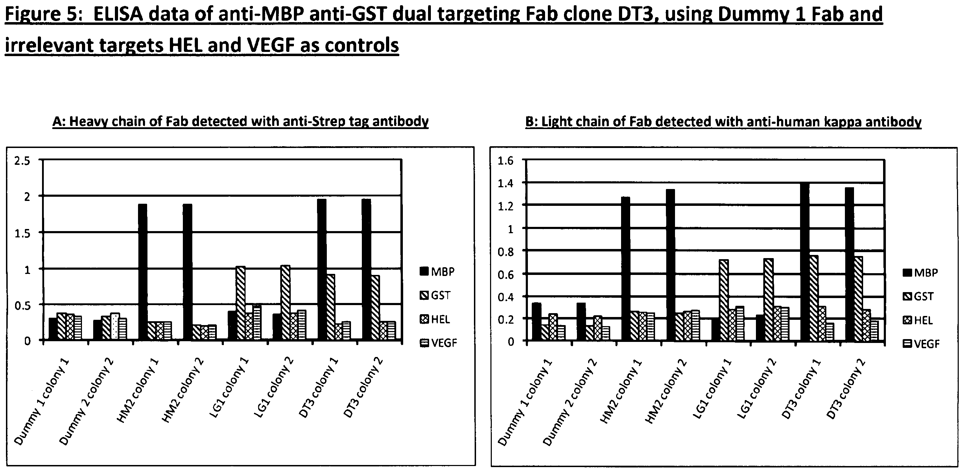

To test the specificity of antibodies selected from Lib1 and Lib2 libraries against one target and the specificity of bi-specific antibodies designed to bind both targets, Enzyme-linked immunosorbent assays (ELISAs) were performed using standard methods. Briefly, Nunc Maxisorp plates were prepared by coating with Streptavidin dissolved in 1x PBS, binding 20 nM of biotinylated targets (GST, MBP, HEL or VEGF) in PBS-T (0.3% Tween-20 dissolved in 1x PBS) and blocking with 5% skimmed milk powder in PBS-T. Thereafter, 50 µl of *E*. *coli* TG1 culture supernatant expressing antibody clones as soluble Fab fragments in microtiter plates were added, followed by detection of bound Fab fragments using goat anti-human kappa light chain polyclonal antibody (Sigma) or mouse anti-Strep tag antibody (IBA) specific for a Strep-II tag fused to the C-terminus of the heavy chain in the soluble Fab expression construct. Secondary antibodies were detected using HRP-labeled tertiary antibodies, ELISAs were developed using TMB substrate (KPL), and signal was quantified using a Victor plate reader from PerkinElmer set to 450 nm. It was found that Dummy 1 Fab secreted into the *E. coli* culture supernatant bound none of the four targets, Fab LG1 (that had been selected from library Lib D1L1) bound only GST, Fab HM2 (that had been selected from library Lib D1H1) bound only MBP, and Fab DT3 (that combined all the target-specific residues found in Fabs LG1 and HM2) bound only GST and MBP. None of the clones bound the control targets HEL or VEGF (Figure 5). Experiments were performed in duplicate using two independent colonies for each Fab.

### EXAMPLE 8: Affinities of parental and bi-specific antibodies

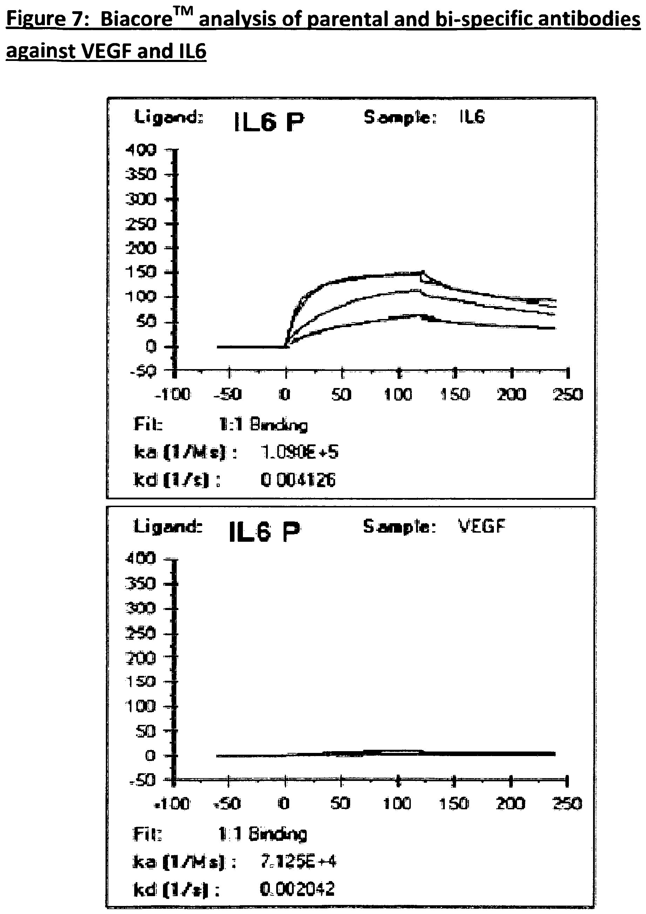

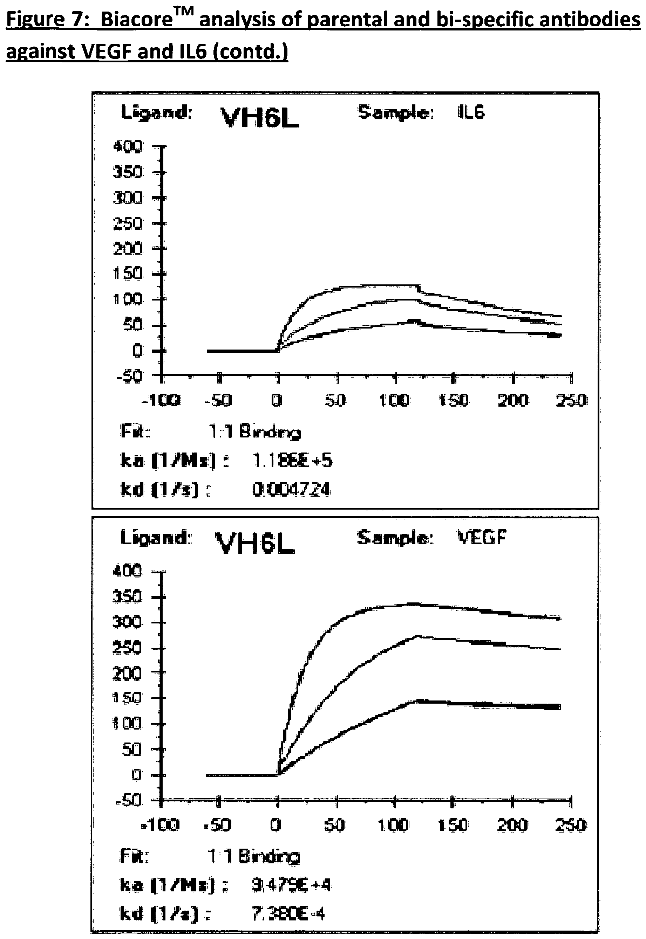

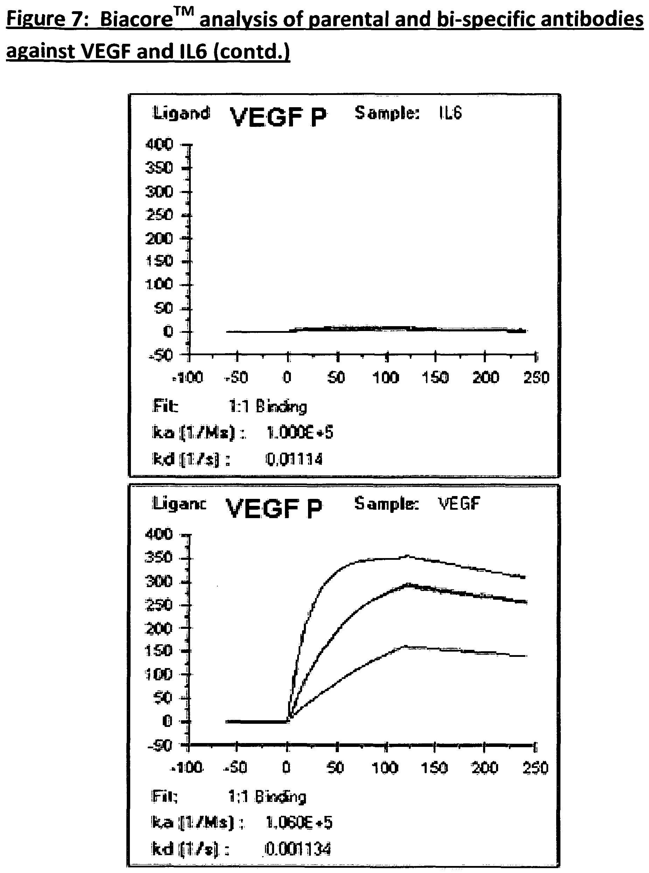

Antibody libraries were selected against human VEGF (Peprotech catalogue number 100-20) and human IL6 (Peprotech catalogue number 200-06). Of the isolated parental antibody clones, IL6P and VEGFP were combined into the bi-specific antibody clone VH6L. The sequence of VH6L is shown in Figure 4, which shows an additional point mutation at amino acid 4 of the light chain. To assess the affinities of parental and bi-specific antibodies, Biacore^{™} analysis was performed in order to analyze the binding behaviour of IL6P, VH6L and VEGFP. For this, an anti-light chain capture antibody was immobilized onto a CM5 chip using amine-coupling, resulting in 12000 RU. Fab fragments were captured to a level of 400-500 RU and a concentration series of IL6 and VEGF, ranging from 0 to 450 nM, was passed over the chip. As depicted in Figure 7, clone IL6P binds to IL6, but not to VEGF, clone VEGFP binds to VEGF, but not to IL6, and the combined clone VH6L binds to both IL6 and VEGF. As shown in Table 1, the affinities to the targets are similar for the parental and bi-specific antibodies. The dissociation constant, KD, is 38 nM and 40 nM for IL6P and VH6L, respectively, and 11 nM and 7.8 nM for VEGFP and VH6L, respectively.

**Table 1. Affinity measurements**

| **Ligand** | **Sample** | **ka** | **Kd** | **KD** |
|---|---|---|---|---|
| IL6P | IL6 | 1.1E+05 | 4.1E-03 | 3.8E-08 |
| VH6L | IL6 | 1.2E+05 | 4.7E-03 | 4.0E-08 |
| VEGFP | IL6 | N/A | N/A | NB |
| IL6P | VEGF | N/A | N/A | NB |
| VH6L | VEGF | 9.5E+04 | 7.4E-04 | 7.8E-09 |
| VEGFP | VEGF | 1.1E+05 | 1.1E-03 | 1.1E-08 |

### EXAMPLE 9: Co-binding of two antigens to the same VH-VL variable region

In order to demonstrate that bi-specific antibodies according to the invention can bind two different antigens simultaneously through the same VH-VL variable region, a Biacore^{™} experiment using the bi-specific antibody clone GH6L specific for human GMCSF and human IL6 was performed. The sequence of GH6L is shown in Figure 4, which shows an additional point mutation at amino acid 4 of the light chain. The antibody was expressed in human IgG1 format using standard mammalian expression vectors bearing GH6L heavy and light chain and signal peptide cDNAs, by transient transfection of HEK293-6E cells. Expressed IgG was affinity-purified using protein A resin. For Biacore^{™} analysis, GMCSF (Peprotech catalogue number 300-03) or an anti-light chain capture antibody was immobilized onto a CM5 chip using amine-coupling, resulting in 4000 RU and 12000 RU immobilized GMCSF and anti-light chain capture antibody, respectively.

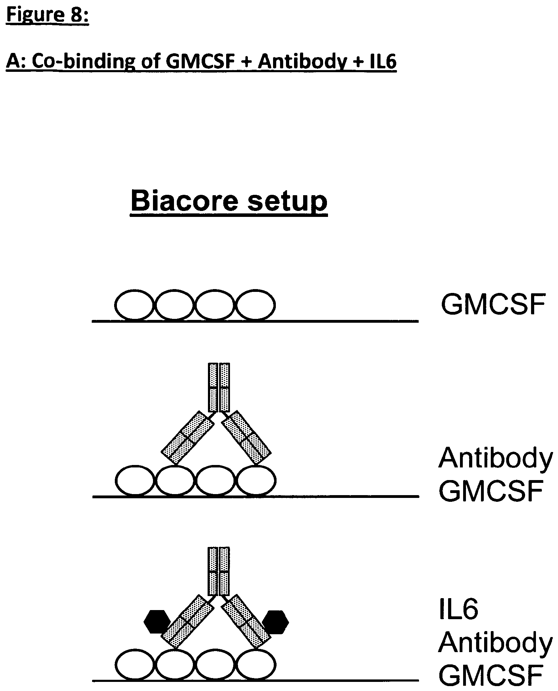

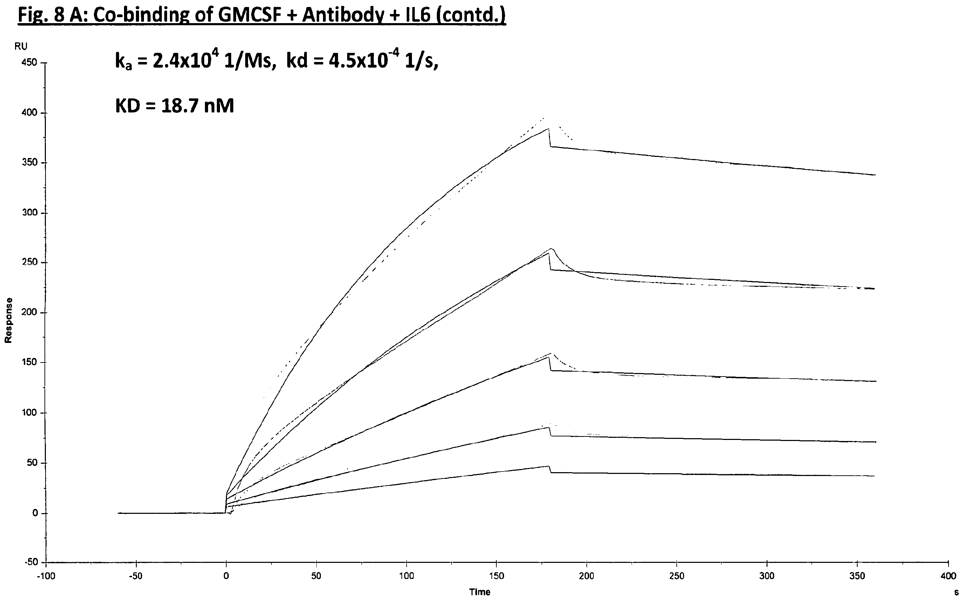

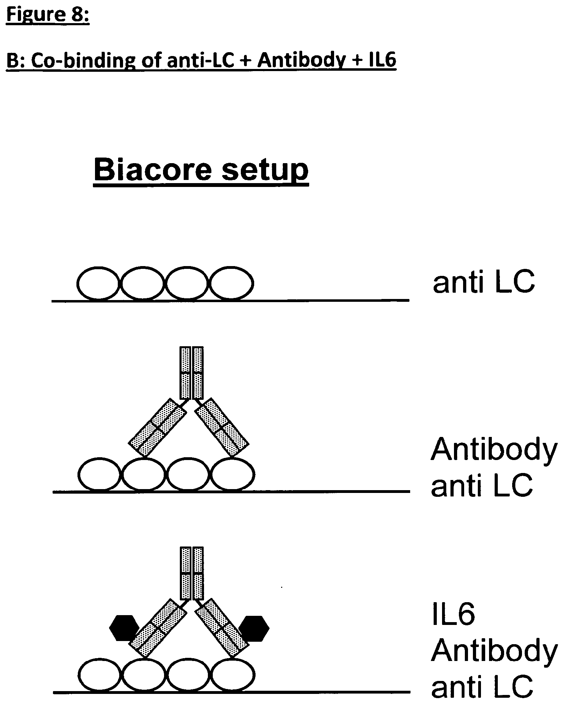

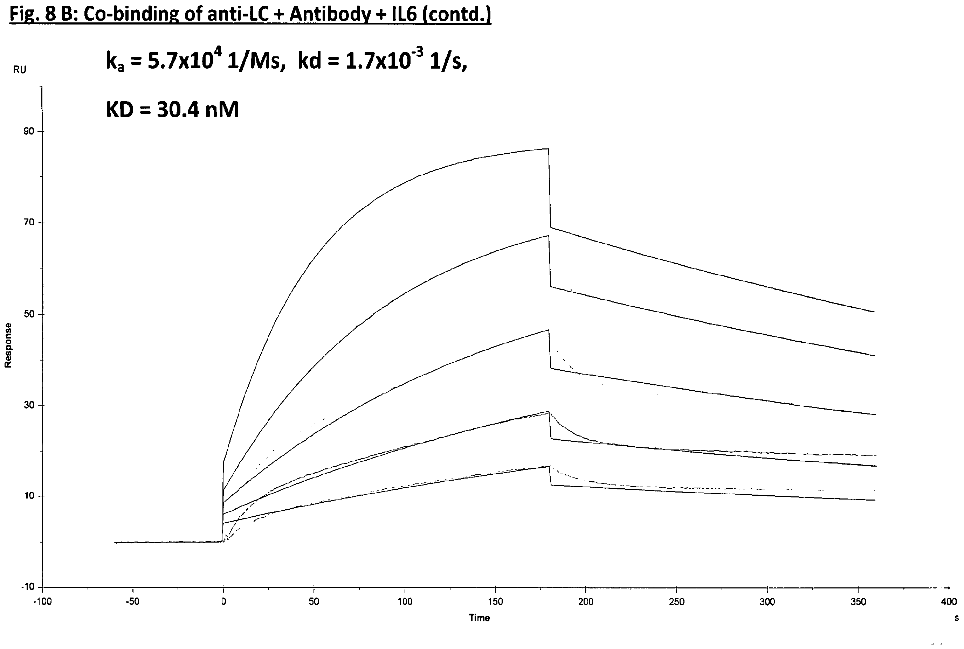

GH6L was captured onto the prepared surfaces, and in each case a concentration series of IL6 (Peprotech catalogue number 200-06) was flown over, and data were analyzed using BIAevaluation software. As can be seen in Figure 8A, GH6L captured onto GMCSF can bind to IL6. A control experiment injecting GMCSF did not give rise to a signal showing that the IL6 binding signal was due to simultaneous binding at the same VH-VL variable region rather than binding of a "free arm" of GH6L not interacting with GMCSF on the chip surface. In Figure 8B, GH6L is captured by the generic anti-light chain capture antibody to measure the IL6 binding affinity of GH6L without the presence of GMCSF. Comparing Figures 8A and 8B, it can be seen that GH6L binds to IL6 with similar affinity regardless of whether GH6L is bound to GMSCF or not.

### EXAMPLE 10: Independent binding behaviour

For several bi-specific antibodies according to the invention, the independent behaviour of the two binding sites could be shown using site-directed mutagenesis of single residues located within the Lib1 or Lib2 binding regions. In one instance, a bi-specific antibody clone directed against Target A and Target B was mutated.

By incorporating a single conservative LCDR3 point mutation H93Y within the Lib1 binding region providing the putative paratope involved in Target A-binding, affinity for Target A was largely abolished, whilst affinity for Target B was left intact.

On the other side, by incorporating a single conservative LCDR2 point mutation W56Y within the Lib2 binding region of that antibody clone providing the putative paratope involved in Target B-binding affinity for Target B was completely abolished whilst affinity for Target A was left intact.

In another instance, a bi-specific antibody clone directed against Target C and Target D was mutated. By incorporating a single conservative LCDR1 point mutation N27D within the Lib1 binding region providing the putative paratope involved in Target C-binding, affinity for Target C was largely abolished, whilst affinity for Target D was left intact.

On the other side, by incorporating a single HCDR1 point mutation L28D or a single LCDR2 point mutation Y56D within the Lib2 binding region of this second antibody clone providing the putative paratope involved in Target D binding, affinity for Target D was abolished whilst affinity for Target C was left intact.

## Claims

1. A bispecific antibody or functional fragment thereof comprising at least one variable binding domain consisting of a heavy chain variable (VH) domain and a light chain variable (VL) domain, wherein said binding domain comprises two paratopes for two unrelated epitopes, wherein (i) binding of each paratope to its epitope does not prevent the simultaneous binding of the other paratope to its respective epitope, and wherein (ii) the first paratope comprises residues from CDR1 and CDR3 of the VL domain and CDR2 of the VH domain, and the second paratope comprises residues from CDR1 and CDR3 of the VH domain and CDR2 of the VL domain.

2. The antibody or functional fragment thereof of claim 1, which is a bispecific antibody, wherein said two unrelated epitopes are present on two different molecules.

3. The antibody or functional fragment thereof of claim 1 or 2, wherein the amount of binding of each paratope to its respective epitope in the simultaneous presence of both epitopes is at least 25% of the amount of binding that is achieved in the absence of the other epitope under otherwise identical conditions.

4. The antibody or functional fragment thereof of claim 3, wherein the amount of binding is at least 50%, particularly at least 75%, and more particularly at least 90%.

5. The antibody or functional fragment thereof of any one of claims 1 to 4 that is a human antibody or functional fragment thereof.

6. The antibody or functional fragment thereof of claim 5 that is based on a human VH3 family heavy chain sequence and a human Vkappa1 family light chain sequence.

7. The antibody or functional fragment thereof of claim 5 that is based on a human VH3 family heavy chain sequence and a human Vlambda1 family light chain sequence.

8. The antibody or functional fragment thereof of any one of claims 1 to 7, wherein the antibody or functional fragment thereof is selected from a single chain Fv fragment, a Fab fragment and an IgG.

9. A nucleic acid sequence encoding the antibody or functional fragment thereof according to any one of claims 1 to 8.

10. A vector comprising the nucleic acid sequence according to claim 9.

11. A host cell, which is an isolated host cell, comprising the nucleic acid sequence according to claim 9 or the vector according to claim 10.

12. A method for generating the antibody or functional fragment thereof of any one of claims 1 to 8, comprising the step of expressing the nucleic acid sequence according to claim 9, or the vector according to claim 10, either in vitro or from an appropriate host cell, which is an isolated host cell, including the host cell according to claim 11.

## Patentansprüche

1. Bispezifischer Antikörper oder funktionelles Fragment davon, umfassend mindestens eine variable Bindungsdomäne, bestehend aus einer variablen Domäne der schweren Kette (VH) und einer variablen Domäne der leichten Kette (VL), wobei die Bindungsdomäne zwei Paratope für zwei nicht verwandte Epitope umfasst, wobei (i) das Binden jedes Paratops an sein Epitop das gleichzeitige Binden des anderen Paratops an sein jeweiliges Epitop nicht verhindert und wobei (ii) das erste Paratop Reste aus CDR1 und CDR3 der VL-Domäne und CDR2 der VH-Domäne umfasst und das zweite Paratop Reste aus CDR1 und CDR3 der VH-Domäne und CDR2 der VL-Domäne umfasst.

2. Antikörper oder funktionelles Fragment davon nach Anspruch 1, der ein bispezifischer Antikörper ist, wobei die beiden nicht verwandten Epitope auf zwei verschiedenen Molekülen vorliegen.

3. Antikörper oder funktionelles Fragment davon nach Anspruch 1 oder 2, wobei das Ausmaß des Bindens jedes Paratops an sein jeweiliges Epitop bei der gleichzeitigen Gegenwart beider Epitope mindestens 25 % des Ausmaßes des Bindens beträgt, das in Abwesenheit des anderen Epitops unter ansonsten identischen Bedingungen erzielt wird.

4. Antikörper oder funktionelles Fragment davon nach Anspruch 3, wobei das Ausmaß des Bindens mindestens 50 %, spezieller mindestens 75 % und noch spezieller mindestens 90 % beträgt.

5. Antikörper oder funktionelles Fragment davon nach einem der Ansprüche 1 bis 4, der/das ein humaner Antikörper oder funktionelles Fragment davon ist.

6. Antikörper oder funktionelles Fragment davon nach Anspruch 5, der/das auf einer Sequenz der schweren Kette der humanen VH3-Familie und einer Sequenz der leichten Kette der humanen Vkappal-Familie basiert.

7. Antikörper oder funktionelles Fragment davon nach Anspruch 5, der/das auf einer Sequenz der schweren Kette der humanen VH3-Familie und einer Sequenz der leichten Kette der humanen Vlambdal-Familie basiert.

8. Antikörper oder funktionelles Fragment davon nach einem der Ansprüche 1 bis 7, wobei der Antikörper oder das funktionelle Fragment davon aus einem Einzelketten-Fv-Fragment, einem Fab-Fragment und einem IgG ausgewählt ist.

9. Nukleinsäuresequenz, die für den Antikörper oder das funktionelle Fragment davon nach einem der Ansprüche 1 bis 8 kodiert.

10. Vektor, umfassend die Nukleinsäuresequenz nach Anspruch 9.

11. Wirtszelle, die eine isolierte Wirtszelle ist, umfassend die Nukleinsäuresequenz nach Anspruch 9 oder den Vektor nach Anspruch 10.

12. Verfahren zum Erzeugen des Antikörpers oder des funktionellen Fragments davon nach einem der Ansprüche 1 bis 8, umfassend den Schritt des Exprimierens der Nukleinsäuresequenz nach Anspruch 9 oder des Vektors nach Anspruch 10, entweder in vitro oder aus einer entsprechenden Wirtszelle, die eine isolierte Wirtszelle, enthaltend die Wirtszelle nach Anspruch 11, ist.

## Revendications

1. Anticorps bispécifique ou fragment fonctionnel de celui-ci comprenant au moins un domaine de liaison variable constitué d'un domaine variable de chaîne lourde (VH) et d'un domaine variable de chaîne légère (VL), dans lequel ledit domaine de liaison comprend deux paratopes pour deux épitopes non apparentés, dans lequel (i) la liaison de chaque paratope à son épitope n'empêche pas la liaison simultanée de l'autre paratope à son épitope correspondant, et dans lequel (ii) le premier paratope comprend des résidus de CDR1 et CDR3 du domaine VL et de CDR2 du domaine VH, et le second paratope comprend des résidus de CDR1 et CDR3 du domaine VH et de CDR2 du domaine VL.

2. Anticorps ou fragment fonctionnel de celui-ci selon la revendication 1, qui est un anticorps bispécifique, dans lequel lesdits deux épitopes non apparentés sont présents sur deux molécules différentes.

3. Anticorps ou fragment fonctionnel de celui-ci selon la revendication 1 ou 2, dans lequel le degré de liaison de chaque paratope à son épitope correspondant en présence simultanée des deux épitopes est d'au moins 25 % du degré de liaison qui est obtenu en l'absence de l'autre épitope dans des conditions par ailleurs identiques.

4. Anticorps ou fragment fonctionnel de celui-ci selon la revendication 3, dans lequel le degré de liaison est d'au moins 50 %, en particulier d'au moins 75 %, et plus particulièrement d'au moins 90 %.

5. Anticorps ou fragment fonctionnel de celui-ci selon l'une quelconque des revendications 1 à 4 qui est un anticorps humain ou un fragment fonctionnel de celui-ci.

6. Anticorps ou fragment fonctionnel de celui-ci selon la revendication 5 qui est basé sur une séquence de chaîne lourde de la famille VH3 humaine et une séquence de chaîne légère de la famille Vkappa1 humaine.

7. Anticorps ou fragment fonctionnel de celui-ci selon la revendication 5 qui est basé sur une séquence de chaîne lourde de la famille VH3 humaine et une séquence de chaîne légère de la famille Vlambda1 humaine.

8. Anticorps ou fragment fonctionnel de celui-ci selon l'une quelconque des revendications 1 à 7, dans lequel l'anticorps ou un fragment fonctionnel de celui-ci est choisi parmi un fragment Fv à chaîne unique, un fragment Fab et une IgG.

9. Séquence d'acide nucléique codant pour l'anticorps ou un fragment fonctionnel de celui-ci selon l'une quelconque des revendications 1 à 8.

10. Vecteur comprenant la séquence d'acide nucléique selon la revendication 9.

11. Cellule hôte, qui est une cellule hôte isolée, comprenant la séquence d'acide nucléique selon la revendication 9 ou le vecteur selon la revendication 10.

12. Procédé pour générer l'anticorps ou un fragment fonctionnel de celui-ci selon l'une quelconque des revendications 1 à 8, comprenant l'étape consistant à exprimer la séquence d'acide nucléique selon la revendication 9, ou le vecteur selon la revendication 10, soit in vitro, soit à partir d'une cellule hôte appropriée, qui est une cellule hôte isolée, incluant la cellule hôte selon la revendication 11.
